# EUROPEAN PATENT APPLICATION

(11) **EP 3 246 833 A1**
(43) Date of publication of application: **22.11.2017**
(21) Application number: 16170322.8
(22) Date of filing: 19.05.2016
(51) Int. Cl.: G06F 19/00

(54) **METHOD AND SYSTEM FOR TRACKING AND GUIDING THINGS AND PERSONS IN A HOSPITAL**

(71) Applicant: Siemens Healthcare GmbH, 91052 Erlangen (DE)
(72) Inventor: Freudenberger, Jörg, 90562 Kalchreuth (DE)

(57) **Abstract**

The invention specifies a method for tracking and guiding of things (5) and persons (4) in a hospital, comprising following steps:
- equipping (100) of things (5) and persons (4) with an unique passive radio frequency transponder (2),
- storing (101) of assignments of the passive radio frequency transponders (2) to the things (5) and the persons (4) in a database (10) of a hospital tracking and guiding computer system (1),
- localizing (102) the passive radio frequency transponders (2) by in-house radio frequency transmitters (3) with an accuracy of less than one meter,
- storing (103) and up-dating the locations of the passive radio frequency transponders (2) in the database (10).

A corresponding system for tracking and guiding of things (5) and persons (4) in a hospital is specified as well.

## Description

### Field of the Invention

The present invention relates to a method and a system for tracking and guiding of things and persons, equipped with passive radio frequency transponders, in a hospital.

### Background of the Invention

In hospitals, patients must be tracked as much as possible in order to be optimally guided and transported to an upcoming treatment or examination. In the future, this task will be even more important because the number of personnel will have to be reduced due to the pressures of cost saving and efficiency in healthcare systems. Treatment and examination facilities should be used continuously with the shortest waiting time for patients as possible.

Furthermore, it should be considered, that patients may have to be carried from one location to another location, patients should be guided from one place to another place and an access of patients should be controlled, e.g. the access of infectious patients to specific areas should be denied or only certain patients are allowed to enter an operating theatre.

Generally speaking, it is expected that patients waiting in marked waiting areas or patient rooms, should be able to independently walk to a destination (e.g. radiology, treatment room) by finding their way based on signs. The transport of patients is often done by hospital staff, e.g. nurses, etc. A central location of patients is not available.

### Summary of the Invention

The objective of the present invention is to provide a method and a system for better handling of things and persons in a hospital.

To solve the problem, the present invention provides a method and a system for tracking and guiding of things and persons in a hospital. Advantageous embodiments are provided in the dependent claims.

According to the invention a central patient management system, the so-called system for tracking and guiding things and persons, is aware of the exact location (= spatial position) of persons (e.g. hospitalized patients or staff) and things (e.g. ultrasound head, x-ray detector) at any time. Therefore, things and persons are equipped with passive radio frequency transponders (e.g. RFID tags) which are localized by in-house (= in the hospital) radio frequency transmitters connected to a hospital tracking and guiding computer of the system. Localization can for example be performed by measuring signal travelling times between the transmitters and the transponders and triangulation. Per definition, a location is a particular point or place in a physical space.

The invention claims a method for tracking and guiding of things and persons in a hospital. The method comprises the steps of:
- equipping things and persons with an unique passive radio frequency transponder,
- storing assignments of the passive radio frequency transponders to the things and the persons in a database of a hospital tracking and guiding computer system,
- localizing the passive radio frequency transponders by in-house radio frequency transmitters with an accuracy of less than one meter and
- storing and up-dating the locations of the passive radio frequency transponders in the database.

The advantage of the invention lies in the automatically determined knowledge of the exact locations of persons and things in a hospital.

In a further embodiment the method comprises matching the locations of the passive radio frequency transponders with a digital map of the hospital by the hospital tracking and guiding computer system.

Map matching is the method of matching recorded coordinates in space to a logical model (e.g. digital map) of the real world.

In a further embodiment the method comprises displaying the locations of the passive radio frequency transponders on the digital map on a computer screen of the central hospital computer.

In a further embodiment the method comprises displaying the locations of passive radio frequency transponders in the digital map on a local screen only for passive radio frequency transponders which are in close vicinity (less than several meters) to the local screen.

Advantageous is that communication with patients and staff is possible without using mobile phones, which use is problematic in certain areas of a hospital.

In a further embodiment the method comprises storing access authorisations to rooms in the hospital and/or of authorisations for opening doors in the hospital for the passive radio frequency transponders of persons in the database.

In a further embodiment the method comprises automatically opening of a door of a room to which access is allowed for a person, if the person is in the vicinity (less than several meters) of that door.

Therefore, the contact of infectious patients with other people can be controlled and watched safely, the access to treatment rooms can be arranged easily and it can be safeguarded that only the right patient is operated on in an operating theatre.

In a further embodiment the method comprises automatically displaying guiding information for the person on a local screen which is in viewing distance of said person (less than several meters) based on the location information of the passive radio frequency transponder of the person in the database.

In a further embodiment the method comprises automatically providing information for the person via a loudspeaker which is in a hearing distance of said person (e.g. in the room where the loudspeaker is mounted) based on the location information of the passive radio frequency transponder of the person in the database.

Furthermore, the invention claims a system for tracking and guiding things and persons in a hospital. The system comprises:
- passive radio frequency transponders, which are attached to things and/or persons in order to uniquely identify a specific thing or a specific person,
- a hospital tracking and guiding computer system comprising at least one database cofigured and programmed for storing assignments of the passive radio frequency transponders to the things and the persons and
- in-house radio frequency transmitters cofigured for localizing the passive radio frequency transponders with an accuracy of less than one meter,
- whereas the hospital tracking and guiding computer system is cofigured and programmed to store and up-date said locations of the passive radio frequency transponders in the database.

In a further embodiment the hospital tracking and guiding computer system is cofigured and programmed to match the locations of the passive radio frequency transponders with a digital map of the hospital.

In a further embodiment the system comprises:
- a computer screen connected to the hospital tracking and guiding computer system,
- whereas the hospital tracking and guiding computer system is cofigured and programmed to display the locations of the passive radio frequency transponders on the digital map on the computer screen.

In a further embodiment the system comprises:
- local screens connected to the hospital tracking and guiding computer system,
- whereas the hospital tracking and guiding computer system is cofigured and programmed to display the locations of passive radio frequency transponders in the digital map on the local screen only for passive radio frequency transponders which are in close vicinity to the local screen.

In a further embodiment the hospital tracking and guiding computer system is cofigured and programmed to automatically display guiding information for a person on the local screen which is in viewing distance of the person, based on the location information of the passive radio frequency transponder of the person in the database.

In a further embodiment the system comprises:
- doors connected to the hospital tracking and guiding computer system cofigured and programmed to automatically open the door to a room to which access is allowed for the person, if said person is in the vicinity of that door,
- whereas the hospital tracking and guiding computer system is cofigured and programmed to store access authorisations to rooms in the hospital and/or authorisations for opening the doors in the hospital for passive radio frequency transponders of the persons in the database.

In a further embodiment the system comprises:
- loudspeakers connected to the hospital tracking and guiding computer system,
- whereas the hospital tracking and guiding computer system is cofigured and programmed to automatically announce information for the person via the loudspeaker which is in a hearing distance of the person, based on the location information of the passive radio frequency transponder of the person in the database.

Further benefits and advantages of the present invention will become apparent after a careful reading of the detailed description with appropriate reference to the accompanying drawings.

### Brief Description of the Drawings

- Fig. 1:: shows a block diagram of a system for tracking and guiding things and persons in a hospital and
- Fig. 2:: shows a flow chart of a method for tracking and guiding things and persons in a hospital.

### Detailed Description of the Invention

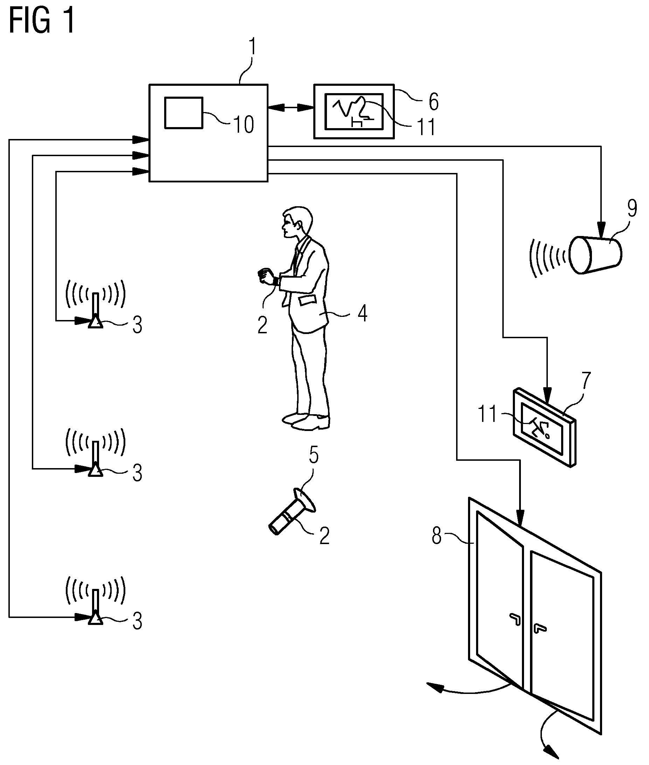

**Fig. 1** shows a block diagram of a system for tracking and guiding things 5 and persons 4 in a hospital. Heart of the system is the hospital tracking and guiding computer 1 which is preferably located in a central office of the hospital. The hospital tracking and guiding computer 1 comprises a data base 10 for storing information related to things 5 and persons 4 in the hospital.

Selected things 5, e.g. medical equipment and medical devices, as well as selected persons 4, e.g. patients in the hospital, are equipped with a unique passive radio frequency transponder 2, e.g. an RFID tag. For example, the patients 4 wear a wristband or a neckband comprising the passive radio frequency transponder 2. In order to uniquely identify the persons 4 or the things 5 a table comprising data of persons and corresponding passive radio frequency transponder IDs and/or a table comprising data of things and corresponding passive radio frequency Ids is stored in the data base 10 of the hospital tracking and guiding computer 1. This is the so-called assignments of the passive radio frequency transponders 2. In addition, permissions to selected rooms for selected persons 4 area stored in the data base 10 as well.

For locations detection, a plurality of radio frequency transmitters 3 is distributed in the hospital such that every passive radio frequency transponder 2 has radio contact to more than one radio frequency transmitter 3 in one room. Via radiolocation, e.g. signal transmission time measurement and triangulation, the exact location of the passive radio frequency transponders 2, and therefore the location of things and persons, can be determined. This location data is tracked in the hospital tracking and guiding computer 1 and matched with a digital map 11 of the hospital building. Hence, the locations of things 5 and persons 4 are known at any time and can be shown on the computer screen 6 of the hospital tracking and guiding computer 1 together with the digital map 11. The location information of things and persons can be used for a variety of location-based applications.

Due to the tracking of persons 4 via their passive radio frequency transponders 2 doors 8 connected to the hospital tracking and guiding computer 1 can be opened automatically for persons 4 with access permission to rooms behind these doors 8. For example, patients 4 are allowed to enter a treatment room or a waiting room for infected patients. On the other hand, doors 8 automatically close if persons 4 without access permission are approaching. All these authorisations and permissions are stored in the data base 10 of the hospital tracking and guiding computer 1.

Local screens 7 are distributed in the hospital in order to show a person 4, who is in viewing distance to any local screens 7, his or her location on the digital map 11 on the local screen 7. For that reason, the local screens 7 are connected to the hospital tracking and guiding computer which transmits this information onto the local screens 7. Only positions and related information for persons 4 who are in the vicinity of the local screens 7 are displayed. The information can comprise guiding and related information for patients in order to reach a desired location in the hospital, e.g. a treatment room or an operating theatre.

Loudspeakers 9 connected to the hospital tracking and guiding computer 1 are spread over the hospital building in order to provide acoustic information to persons 4 who are in the vicinity of the loudspeakers 9. Only information concerning a specific person 4 is transmitted via the loudspeaker 9 if that specific person 4 is in hearing distance to the loudspeaker 9. For example, the voice information can comprise guiding information for patients or emergency information for hospital staff.

Preferably, the hospital tracking and guiding computer 1 has an interface to a patient data management system. Hence, the hospital tracking and guiding computer 1 knows where a patient has to go or what treatments are following next. Included is information about the physical situation of the patient and his or her general condition.

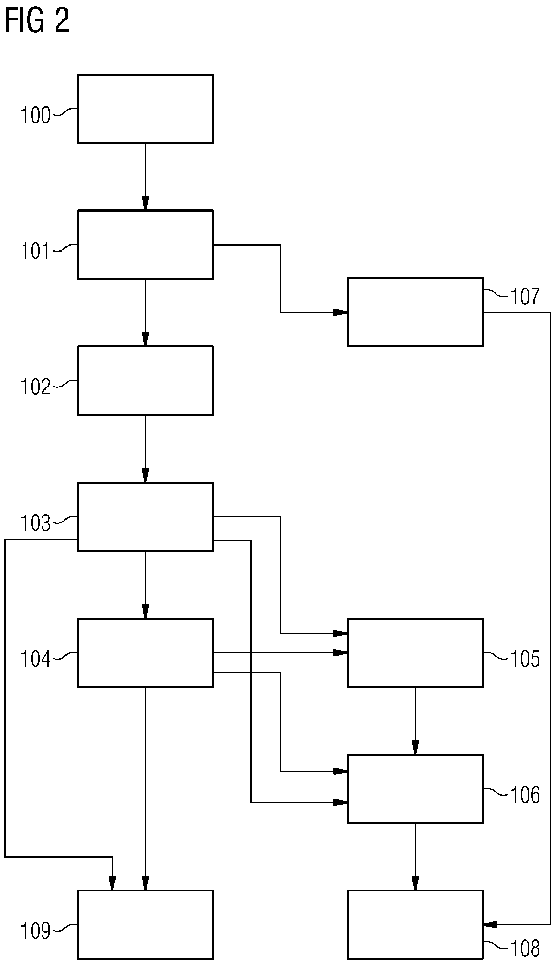

**Fig. 2** shows a flow chart of a method for tracking and guiding of things 5 and persons 4 in a hospital. In step 100 of the method persons 4 and things 5 in a hospital are equipped with a passive radio frequency transponder 2. In step 101 the assignments of persons 4 and things 5 to a unique passive radio frequency transponder 2 are stored in a data base 10 of a hospital tracking and guiding computer 1. Via radio frequency transmitters 3 which are spread across the hospital building the location of every passive radio frequency transponder 2 is determined. In step 103 this location information is stored and up-dated in the data base 10. The access authorization for selected persons 4 to rooms and therefore the permission to open room-related doors 8 is stored in the data base 10 in step 107.

In step 104 the locations of the passive radio frequency transponders 2 are matched with a digital map 11 of the hospital building. In said digital map 11 all rooms, doors, walls, etc. are drawn. In step 105 the locations of the passive radio frequency transponders 2 in relation to the digital map 11 are displayed on a computer screen 6 of the hospital tracking and guiding computer 1. On local screen 7, which are spread all over the hospital building, information for persons 4, who are in viewing distance to a local screen 7, is displayed in step 106.

In step 108 a door 8 is opened automatically if a person 4 permitted to enter a room behind said door 8 is in opening distance to said door. In step 109 person-related information is announced over a loudspeaker 9 if said person 4 is in hearing distance to said loudspeaker 9 and voice information is available.

Although the invention has been explained in relation to its preferred embodiment(s) as mentioned above, it is to be understood that many other possible modifications and variations can be made without departing from the scope of the present invention. It is, therefore, contemplated that the appended claim or claims will cover such modifications and variations that fall within the true scope of the invention.

### List of Reference Signs

- 1: hospital tracking and guiding computer system
- 2: passive radio frequency transponder
- 3: radio frequency transmitter
- 4: person
- 5: thing
- 6: computer screen
- 7: local screen
- 8: door
- 9: loudspeaker
- 10: data base
- 11: digital map of the hospital

- 100: equipping with passive radio frequency transponders 2
- 101: storing of assignments
- 102: localizing a passive radio frequency transponder 2
- 103: storing and up-dating the locations
- 104: map-matching
- 105: displaying on a computer screen 6
- 106: displaying on a local screen 7
- 107: storing of access authorisations
- 108: opening/closing door 8
- 109: providing acoustic information

## Claims

1. Method for tracking and guiding of things (5) and persons (4) in a hospital, **characterised by:**
- equipping (100) things (5) and persons (4) with an unique passive radio frequency transponder (2),
- storing (101) assignments of the passive radio frequency transponders (2) to the things (5) and the persons (4) in a database (10) of a hospital tracking and guiding computer system (1),
- localizing (102) the passive radio frequency transponders (2) by in-house radio frequency transmitters (3) with an accuracy of less than one meter,
- storing (103) and up-dating the locations of the passive radio frequency transponders (2) in the data base (10).

2. Method according to claim 1, **characterised by:**
- matching (104) the locations of the passive radio frequency transponders (2) with a digital map (11) of the hospital by the hospital tracking and guiding computer system (1).

3. Method according to claim 2, **characterised by:**
- displaying (105) the locations of the passive radio frequency transponders (2) in the digital map (11) on a computer screen (6) of the central hospital computer system (1).

4. Method according to claim 2 or claim 3, **characterised by:**
- displaying (106) the locations of passive radio frequency transponders (2) on the digital map (11) on a local screen (7) only for passive radio frequency transponders (2) which are in close vicinity to the local screen (7).

5. Method according to any of the previous claims, **characterised by:**
- storing (107) access authorisations to rooms in the hospital and/or of authorisations for opening doors (8) in the hospital for the passive radio frequency transponders (1) of selected persons in the database (10).

6. Method according to claim 5, **characterised by:**
- automatically opening (108) a door (8) of a room to which access is allowed for the selected person (4), if the person (4) is in the vicinity of said door (8).

7. Method according to any of the previous claims, **characterised by:**
- automatically displaying (106) of guiding information for a selected person (4) on a local screen (7) which is in viewing distance of the person (4) based on the location information of the passive radio frequency transponder (2) of said person (4) in the database (10).

8. Method according to any of the previous claims, **characterised by:**
- automatically announcing (109) information for a selected person (4) via a loudspeaker (9) which is in a hearing distance of said person (4) based on the location information of the passive radio frequency transponder (2) of said person (4) in the database (10).

9. System for tracking and guiding things (5) and persons (4) in a hospital, comprising:
- passive radio frequency transponders (2), which are attached to things (5) and/or persons (4) in order to uniquely identify a specific thing (5) or a specific person (4),
- a hospital tracking and guiding computer system (1) comprising at least one database (10) cofigured and programmed for storing assignments of the passive radio frequency transponders (2) to said things (5) and said persons (4) and
- in-house radio frequency transmitters (3) cofigured for localizing the passive radio frequency transponders (2) with an accuracy of less than one meter,
- whereas the hospital tracking and guiding computer system (1) is cofigured and programmed to store and up-date the locations of the passive radio frequency transponders (2) in the database (10).

10. System according to claim 9,
whereas the hospital tracking and guiding computer system (1) is cofigured and programmed to match the locations of the passive radio frequency transponders (2) with a digital map (11) of the hospital.

11. System according to claim 10, comprising:
- a computer screen (6) connected to the hospital tracking and guiding computer system (1),
- whereas the hospital tracking and guiding computer system (1) is cofigured and programmed to display the locations of the passive radio frequency transponders (2) on the digital map (11) on the computer screen (6).

12. System according to claim 10 or 11, comprising:
- local screens (7) connected to the hospital tracking and guiding computer system (1),
- whereas the hospital tracking and guiding computer system (1) is cofigured and programmed to display the locations of passive radio frequency transponders (2) in the digital map (11) on the local screen (7) only for passive radio frequency transponders (2) which are in close vicinity to the local screen (7).

13. System according to claim 12,
whereas the hospital tracking and guiding computer system (1) is cofigured and programmed to automatically display guiding information for a person (4) on the local screen (7) which is in viewing distance of said person (4), based on the location information of the passive radio frequency transponder (2) of said person (4) in the database (10).

14. System according to one of the claims 9 to 13, comprising:
- doors (8) connected to the hospital tracking and guiding computer system (1) cofigured and programmed to automatically open a door to a room to which access is allowed for a person (4), if the person (4) is in the vicinity of that door (8),
- whereas the hospital tracking and guiding computer system (1) is cofigured and programmed to store for passive radio frequency transponders (2) of persons (4) access authorisations to rooms in the hospital and/or authorisations for opening the doors (8) in the hospital in the database (10).

15. System according to one of the claims 9 to 14, comprising:
- loudspeakers (9) connected to the hospital tracking and guiding computer system (1),
- whereas the hospital tracking and guiding computer system (1) is cofigured and programmed to announce information for the person (4) via the loudspeaker (9) which is in a hearing distance of said person (4), based on the location information of the passive radio frequency transponder (2) of said person (4) in the database (10).
